# EUROPEAN PATENT APPLICATION

(11) **EP 4 796 626 A1**
(43) Date of publication of application: **26.08.2026**
(21) Application number: 25159753.0
(22) Date of filing: 24.02.2025
(51) Int. Cl.: C12M 1/32, C12M 1/12, C12M 1/34, C12M 1/36

(54) **REACTOR FOR THE GENERATION OF CONCENTRATION GRADIENTS OF FLUIDS**

(71) Applicant: Charité - Universitätsmedizin Berlin Körperschaft des öffentlichen Rechts, 10117 Berlin (DE); Freie Universität Berlin, Körperschaft des öffentlichen Rechts, 14195 Berlin (DE)
(72) Inventor: Lommel, Michael, 10585 Berlin (DE); Becke, Sophie, 10625 Berlin (DE); Rolff, Jens, 14195 Berlin (DE)
(74) Representative: Hertin und Partner Rechts- und Patentanwälte PartG mbB

(57) **Abstract**

The invention relates to a gradient reactor for generating a concentration gradient, comprising: a) a first layer comprising i) at least one fluid inlet for an injection of at least one fluid, ii) at least one dilutant inlet for an injection of a dilutant, and iii) at least one outlet for an ejection of the at least one fluid, b) a second layer, c) a third layer suitable for culturing microorganisms or cells, wherein a concentration gradient is formable in the second layer, and the concentration gradient is transferable from the second layer to the third layer. The invention further relates to a system comprising i) the gradient reactor according to any one of the preceding claims, wherein the gradient reactor optionally further comprises pumps, preferably micropumps, for automated injection of the at least one fluid and the dilutant, ii) a camera system, and iii) a computer program comprising instructions, optionally wherein the instructions, when the program is executed by a computer, cause the computer to analyze the growth, proliferation and/or survival of the microorganisms or cells. The invention also relates to the use of the gradient reactor according to any one of the preceding claims, for the generation of overlapping stationary or dynamic concentration gradients, for analyzing the efficacy or effect of different biologically active compounds, for assessing possible combinatory therapies, for assessing the probability of future resistance to the different biologically active compounds, or for analyzing pharmacokinetics. The invention also relates to a method for generating one or more concentration gradients, comprising: i) providing a gradient reactor according to the invention, or a system according to the invention, ii) simultaneously injecting the dilutant and the at least one fluid, into the at least one dilutant inlet and the at least one fluid inlet, respectively, iii) generating one or more concentration gradients in the second layer, iv) wherein the one or more concentration gradients transfer from the second layer to the third layer, wherein the third layer is suitable for culturing microorganisms or cells.

## Description

The invention is in the field of reactors used for the generation of (overlapping) concentration gradients of fluids comprising biologically active compounds, and for analyzing the efficacy and/or effect of said biologically active compounds on microorganisms, particularly pathogenic microorganisms, or cells, e.g. tumor cells.

### BACKGROUND OF THE INVENTION

The significant increase of antibiotic-resistant bacteria represents one of the greatest medical challenges worldwide.

Several different methods for Antimicrobial Susceptibility Testing (AST) are known in the art. Conventional, phenotypical, (proliferation-based) AST approaches are performed on pure, cultured isolates. The gold standard are dilution approaches in liquid media and agar. Further frequently used methods are the Kirby-Bauer (KB) disk diffusion test and concentration gradient test strips such as the Epsilometer test (HATTAB, S. er al. Rapid Phenotypic and Genotypic Antimicrobial Susceptibility Testing Approaches for Use in the Clinical Laboratory. Antibiotics (Basel). 2024 Aug 22;13(8):786).

For the KB disk diffusion test, platelets soaked with antibiotics are placed onto inoculated agar plates and the susceptibility is determined based on the size of the inhibition zone around the platelets.

Epsilometer tests function similarly. Here, strips with predefined gradients of an antimicrobial substance are placed onto inoculated agar plates. Based on the resulting inhibition zone, the minimal inhibitory concentration (MIC) can then be read from a scale on the strip.

For the aforementioned test systems, commercial solutions exist already, some of which can also be carried out automatically (VAN BELKUM, A. et al. Innovative and rapid antimicrobial susceptibility testing systems. Nat Rev Microbiol. 2020 May;18(5):299-311).

Nevertheless, the required work effort and cost expenditure and the time span until the result is available are still critical parameters, such that the focus also lies on the development of faster methods ("Rapid AST") since some time. Besides phenotypic approaches, genotypic approaches, which are based on detection of specific genes that convey resistance, play an important role. Several "Rapid AST"-based methods reached already commercial use and are approved for use in clinical diagnostics; further approaches are in the development pipeline of different companies (HATTAB, S. Rapid Phenotypic and Genotypic Antimicrobial Susceptibility Testing Approaches for Use in the Clinical Laboratory. Antibiotics (Basel). 2024 Aug 22;13(8):786; VAN BELKUM, A. Innovative and rapid antimicrobial susceptibility testing systems. Nat Rev Microbiol. 2020 May;18(5):299-311; NEEDS, S.H. et al. Challenges in Microfluidic and Point-of-Care Phenotypic Antimicrobial Resistance Tests. Front. Mech. Eng., 15 September 2020, Sec. Micro- and Nanoelectromechanical Systems, Volume 6 - 2020).

Due to the increasing problem of antimicrobial resistance, improved systems for susceptibility testing address a major medical need and have a high market potential.

Current systems often require 5 to 7 days to select an appropriate drug, e.g. an antifungal drug. Hence, therapies often can only slowly be initiated or changed, which contributes to high mortality rates.

Therefore, further improved systems are needed, which can quickly generate and adapt concentration gradients.

This would be particularly advantageous for rapid susceptibility and combination therapy testing, as well as to predict resistance development in order to improve diagnostics and therapy guidance so that medicaments, such as antibiotics, can be employed in a more targeted and economic way.

### SUMMARY OF THE INVENTION

The present invention addresses the above need by providing a reactor that can generate one or more compound concentration gradients within, for example, several minutes to a few, e.g. 2, or within 24, hours. The concentration gradients can be stationary or dynamic and it can be changed from a stationary to a dynamic concentration gradient generation and *vice versa.* Moreover, due to the way the concentration gradients are generated, a plurality of different concentrations and concentration combinations of different compounds can be tested simultaneously. The present invention enables the continuous observation of the evolutive adaptation of microorganisms and cells. Thus, a fast prediction of the current and future effectiveness of the respective (e.g. antibiotic, antifungal, etc.) therapy can be determined.

The invention relates to a gradient reactor for generating a concentration gradient, comprising:
- a first layer comprising
   i) at least one fluid inlet for an injection of at least one fluid,
   ii) at least one dilutant inlet for an injection of a dilutant, and
   iii) at least one outlet for an ejection of the at least one fluid,
- a second layer,
- a third layer suitable for culturing microorganisms or cells,
wherein a concentration gradient is formable in the second layer, and the concentration gradient is transferable from the second layer to the third layer.

In embodiments, the concentration gradient is formed in the second layer, and the concentration gradient is transferred from the second layer to the third layer.

In embodiments, the second layer comprises a material selected from the group consisting of i) a porous material, such as a glass filter, ii) stainless steel, iii) 3D-printed material, iv) a material comprising microchannels, and v) combinations thereof.

In embodiments, the third layer has a thickness of 0.05-10 mm, preferably 0.1-5 mm.

In embodiments, each of the at least one fluids comprises a different biologically active compound, wherein the different biologically active compounds influence or are suspected to influence growth, proliferation and/or survival of microorganisms or cells, preferably wherein the third layer comprises the microorganisms or cells.

In embodiments, the different biologically active compounds are selected from the group consisting of
i) antimicrobial agents, such as antibiotic, antiviral, antifungal, or antiparasitic agents,
ii) biocides,
iii) stressors, such as salt,
   and
iv) drugs, such as chemotherapeutic agents.

In embodiments, the concentration gradient is transferable from the second layer to the third layer by diffusion.

In embodiments, the dilutant is selected from the group consisting of water and medium.

In embodiments, at least a portion of the at least one fluid inlet is arranged substantially perpendicularly to a surface of the second layer and towards a center of the surface of the second layer.

In embodiments, the at least one fluid inlet is four fluid inlets, wherein the ends of said fluid inlets, through which the fluids are ultimately injected into the reactor, are arranged to form the corners of a square.

In embodiments, at least a portion of one of the at least one dilutant inlets is arranged between the end of the at least one fluid inlet through which the fluid is ultimately injected into the reactor, and the at least one outlet.

In embodiments, the concentration gradient is a stationary or dynamic concentration gradient.

The invention further relates to a system comprising
- the gradient reactor according to the invention, wherein the gradient reactor optionally further comprises pumps, preferably micropumps, for automated injection of the at least one fluid and the dilutant,
- a camera system, and
- a computer program comprising instructions, optionally wherein the instructions, when the program is executed by a computer, cause the computer to analyze the growth, proliferation and/or survival of the microorganisms or cells.

In embodiments, the system may comprise an integrated scale for displaying the effective concentration of the compound(s) and mixtures of compounds. In embodiments, said integrated scale replaces the camera (system).

In embodiments, the analysis of the growth, proliferation and/or survival of the microorganisms or cells comprises the computation of the effective concentration of the different biologically active compounds.

The invention further relates to a use of the gradient reactor, system or methods according to the invention, for the generation of overlapping stationary or dynamic concentration gradients, for analyzing the efficacy or effect of different biologically active compounds, for assessing possible combinatory therapies, for assessing the probability of future resistance to the different biologically active compounds, or for analyzing pharmacokinetics.

In some embodiments, the use may be for generating microorganisms, which have a high salt resistance, and which can, for instance, be used for biogas production from algae.

The invention further relates to a method for generating one or more concentration gradients, comprising:
- providing a gradient reactor according to the invention, or a system according to the invention,
- simultaneously injecting the dilutant and the at least one fluid, into the at least one dilutant inlet and the at least one fluid inlet, respectively,
- generating one or more concentration gradients in the second layer,
- wherein the one or more concentration gradients transfer from the second layer to the third layer, wherein the third layer is suitable for culturing microorganisms or cells.

In embodiments, the one or more concentration gradients transfer from the second layer to the third layer, wherein microorganisms or cells are cultured on or in the third layer.

### DETAILED DESCRIPTION OF THE INVENTION

The invention relates to a gradient reactor for generating a concentration gradient, comprising:
- a first layer comprising
   i) at least one fluid inlet for an injection of at least one fluid,
   ii) at least one dilutant inlet for an injection of a dilutant, and
   iii) at least one outlet for an ejection of the at least one fluid,
- a second layer,
- a third layer suitable for culturing microorganisms or cells,
wherein a concentration gradient is formable in the second layer, and the concentration gradient is transferable from the second layer to the third layer.

In embodiments, the concentration gradient is formed in the second layer, and the concentration gradient is transferred from the second layer to the third layer.

In embodiments, not only one concentration gradient, but at least one, e.g. one or two or three or four concentration gradients are formable or formed in the second layer and transferable or transferred from the second layer to the third layer.

### First layer

The first layer comprises at least one fluid inlet into which a fluid, which preferably comprises a biologically active compound of interest, can be injected.

In preferred embodiments, at least a portion of said at least one fluid inlet is arranged substantially perpendicularly to a surface of the second layer and towards a center of the surface of the second layer.

In preferred embodiments, the reactor comprises four fluid inlets.

In embodiments, the fluid inlets and/or dilutant inlets according to the invention are tubes or channels or similar structures connectable or connected to injection means, which may comprise, but are not limited to, pumps, such as micropumps, and fluid containers, such as syringes, adapted for fluid and/or dilutant injection.

The injection means according to the invention can be any means, which can accommodate a fluid according to the invention and can inject it into the reactor according to the invention. Hence, multiple injection means are required, at least one for each fluid and at least one for the dilutant.

In embodiments, the gradient is generated by microfluidics.

In preferred embodiments, the at least one fluid inlet is four fluid inlets, wherein the ends of said fluid inlets, through which the fluids are ultimately injected into the reactor, are arranged to form the corners of a square.

In said arrangement, a first fluid comprising a first compound according to the invention can be injected into a first of said fluid inlets, a second fluid comprising a second compound according to the invention can be injected into a second of said fluid inlets, which is arranged opposite of the first fluid inlet in said "square", and the same or two different dilutions or mixtures of the first and second fluid comprising the first and second compound, respectively, can be injected into the other two of said fluid inlets. One, two, three, or four of said fluids can be injected into one, two, three, or four of said fluid inlets, respectively. The aforementioned "dilution or mixture" shall mean a fluid which comprises a combination of the first and the second compound. The concentration of the first and the second compound is predetermined, but can be adjusted, depending on which concentrations are of interest to be tested. For example, the first compound may be mixed with the second compound in such a way that the mixture comprises 10% of the undiluted first compound and 90% of the undiluted second compound. Alternatively, they could be mixed to result in an equimolar mixture, or they are further diluted to a desired degree by using a dilutant. That way, any desired concentration of the first and second compound can be achieved. The skilled person knows how to mix and optionally dilute the fluids comprising the compounds in order to achieve the desired biologically effective end concentrations that shall be tested.

In said arrangement, the ends of a first dilutant inlet, through which the dilutant ultimately is injected into the reactor, may be arranged as a first torus-shaped structure, which may, in embodiments, be arranged substantially parallel to a surface of the second layer. In embodiments, the ends of the first dilutant inlet may additionally comprise one or two spot-shaped structures in said first torus-shaped structure to facilitate injection of the dilutant into the first torus-shaped structure from said spot-shaped structures.

In said arrangement, the ends of a first outlet, through which the fluid(s) ultimately is/are ejected from or drain off the reactor, may, in embodiments, be arranged as a second torus-shaped structure, which encircles the first torus-shaped structure, and which may, in embodiments, be arranged substantially parallel to a surface of the second layer. In certain embodiments, the ends of the first outlet may additionally comprise one or two spot-shaped structures in said second torus-shaped structure to facilitate the ejection or drainage of the fluid(s).

In said arrangement, the ends of a second dilutant inlet, through which the dilutant ultimately is injected into the reactor, may be arranged as a third torus-shaped structure, which encircles the second torus-shaped structure, and which may, in embodiments, be arranged substantially parallel to a surface of the second layer. In embodiments, the ends of the second dilutant inlet may additionally comprise one or two spot-shaped structures in said third torus-shaped structure to facilitate injection of the dilutant into the third torus-shaped structure from said spot-shaped structures.

In alternative embodiments, all torus-shaped structures can be replaced by tube-shaped structures extending from the bottom of the first layer towards the second layer, thus ending in the aforementioned spot-shaped structures (top view).

In embodiments, at least a portion of one or more of the at least one fluid inlets, the at least one dilutant inlets, and/or the at least one outlet is arranged substantially perpendicularly to a surface of the second layer.

In embodiments, the at least one fluid inlet is one, two, three , four, five, six, seven, eight, nine, ten, eleven, twelve, or more fluid inlets.

### Second layer

In the second layer, the fluids (if more than one is used) and, optionally, their dilutions or mixtures, are mixed. In embodiments, the structure of the second layer enables an active mixing of the fluids (if more than one is used) and, optionally, their dilutions or mixtures. In preferred embodiments, the mixing is done by convection.

In embodiments, the second layer comprises a material selected from the group consisting of i) a porous material, such as a glass filter or a porous filter made of another material, ii) stainless steel, iii) 3D-printed material, iv) a material comprising microchannels, v) a mesh structure, and vi) combinations thereof.

### Third layer

The third layer according to the invention is suitable for culturing microorganisms or cells. In embodiments, the third layer comprises culture or growth medium for microorganisms or cells.

In embodiments, the culture medium or growth medium comprises a solid, liquid, or semi-solid medium configured to support the growth and/or proliferation of a population of microorganisms or cells. Each microorganism or cell may require a different type of culture or growth medium. Fungi, for instance, are often cultured in agar and cellulose. The use of a liquid culture or growth medium may, however, require a well-damped system, so that the liquid cannot be affected, impaired or intermixed by external influences during or after the gradient formation.

In embodiments, the third layer comprises microorganisms or cells. In embodiments, the microorganisms or cells can be obtained from a sample of a subject. Said sample may be a tissue, blood, plasma, serum, urine, cerebrospinal fluid, lung, skin, gastrointestinal, stool sample or any other sample obtained from a subject. In embodiments, the sample comprises pathogenic bacteria, viruses, fungi, oomycetes, plasmodium, parasites, and/or combinations thereof. Alternatively or additionally, the sample comprises non-pathogenic bacteria, viruses, fungi, oomycetes, plasmodium, parasites, and/or combinations thereof.

In embodiments, the microorganisms or cells are inoculated on or are cultured in the culture or growth medium. The microorganisms or cells grow against the concentration gradients of the biologically active compounds and their combinations in the overlapping area. In embodiments, the bacteria can be tracked in real-time. In some embodiments, the microorganisms or cells comprise detectable labels, such as fluorescent markers or proteins. In additional or alternative embodiments, areas free or substantially free of, or comprising detectably less microorganisms or cells can be detected, for example, visually by eyesight or by a camera. Only resistant microorganisms or cells will migrate towards the sources with highest concentrations of the biologically active compounds. Hence, in embodiments, the spatial distribution of the microorganisms or cells enables the analysis and identification of optimal concentrations, concentration ratios, the minimum inhibitory concentration, and/or the speed of resistance evolution with respect to the (different) biologically active compound(s) or combinations of different biologically active compounds.

In embodiments, microorganisms or cells with known treatment and/or resistance history, which were obtained from a subject, are compared to the predictions of resistance evolution, efficacy or effect results of the (different) biologically active compound(s) and/or the pharmacokinetics of the present invention.

In embodiments, appropriate and likely effective treatment can be initiated, or treatment can be adjusted, based on the results of the analysis. Hence, the present invention enables a personalized and fast treatment guidance.

Fungal infections, for example, are often neglected, although about 6.5 million acute, life-threatening fungal infections exist every year worldwide with about 3.8 million deaths. Since the present invention enables fast (within 24 hours) and personalized treatment guidance, the present invention may advantageously help to decrease mortality. It is additionally advantageous, that the reactor according to the invention can be fully automated and/or standardized.

In embodiments, the therapy guidance may comprise information on effective concentrations of a biologically active compound, or of combinations of biologically active compounds in order to provide an optimal therapy.

In embodiments, the therapy guidance may comprise an initiation or adjustment of a therapy based on the effective concentrations of a biologically active compound, or of combinations of biologically active compounds.

In embodiments, the third layer has a thickness of 0.05-10 mm, preferably 0.1-5 mm, more preferably 0.3-3 mm, even more preferably 0.5-1.5 mm.

In embodiments, the second layer is sandwiched by the first layer and the third layer. In embodiments, the first layer is below the second layer. In embodiments, the first layer is below the second layer and the third layer is above the second layer.

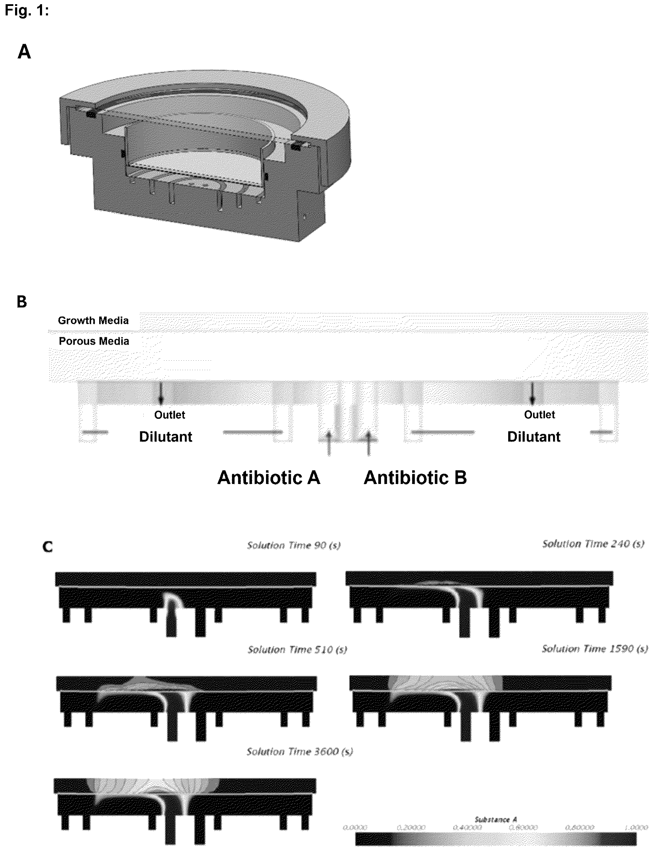

In some embodiments, the third layer is optional (see also **Fig. 1C**). In such an embodiment, the gradient is formed in the second layer, e.g. by convection, but no microorganisms or cells are tested and no diffusion into a third layer occurs.

Hence, the invention also relates to a gradient reactor for generating a concentration gradient, comprising:
- a first layer comprising
   i) at least one fluid inlet for an injection of at least one fluid,
   ii) at least one dilutant inlet for an injection of a dilutant, and
   iii) at least one outlet for an ejection of the at least one fluid,
- a second layer,
- optionally a third layer suitable for culturing microorganisms or cells,
wherein a concentration gradient is formable in the second layer, and optionally wherein the concentration gradient is transferable from the second layer to the third layer.

### Fourth layer

In optional embodiments, the reactor further comprises a fourth layer between the second layer and the third layer, preferably configured to avoid contamination of the reactor with the microorganisms or cells, or with metabolic products from the microorganisms or cells, or with the culture and/or growth medium of the microorganisms or cells, or combinations thereof.

In embodiments, said fourth layer is or comprises a semi-permeable membrane, for example a removable insert comprising a semi-permeable membrane. Said semi-permeable membrane is configured to enable transfer of the concentration gradient (and thus of the fluid(s)) from the second layer to the third layer, while preventing the transfer of microorganisms or cells, or metabolic products from the microorganisms or cells, or the culture and/or growth medium of the microorganisms or cells, or combinations thereof, to the second layer.

In embodiments, the reactor further comprises a lid and a glass plate on top of the third, or, if present, the fourth layer.

In embodiments, the reactor further comprises a gas inlet and/or outlet for regulation of O₂ and/or CO₂ concentrations.

In embodiments, the reactor further comprises an integrated temperature control module.

In embodiments, the gradient reactor further comprises pumps, preferably micropumps, for automated injection of the at least one fluid and the dilutant, and optionally for ejection of the at least one fluid and the dilutant.

In embodiments, the reactor can be used for simultaneous testing of the efficacy of at least two antifungals in a patient sample in a single process step.

In embodiments, dynamically overlapping drug or compound gradients are generated within two hours.

The invention further relates to a method for generating one or more concentration gradients, comprising:
- providing a gradient reactor according to the invention, or a system according to the invention,
- simultaneously injecting the dilutant and the at least one fluid, into the at least one dilutant inlet and the at least one fluid inlet, respectively,
- generating one or more concentration gradients in the second layer,
- optionally, wherein if a third layer is present, the one or more concentration gradients transfer from the second layer to the third layer, wherein the third layer is suitable for culturing microorganisms or cells.

The invention further relates to a method for generating one or more concentration gradients, comprising:
- providing a gradient reactor according to the invention, or a system according to the invention,
- simultaneously injecting the dilutant and the at least one fluid, into the at least one dilutant inlet and the at least one fluid inlet, respectively,
- generating one or more concentration gradients in the second layer,
- wherein the one or more concentration gradients transfer from the second layer to the third layer, wherein the third layer is suitable for culturing microorganisms or cells.

In embodiments, the one or more concentration gradients transfer from the second layer to the third layer, wherein the third layer is not only suitable for culturing microorganisms or cells, but actually comprises microorganisms or cells.

In embodiments, the at least one fluid can be one, two, three, four, five, six, seven, eight, nine, ten, eleven, twelve, or more fluids.

In embodiments, each fluid or combination or mixture is injected into another fluid inlet.

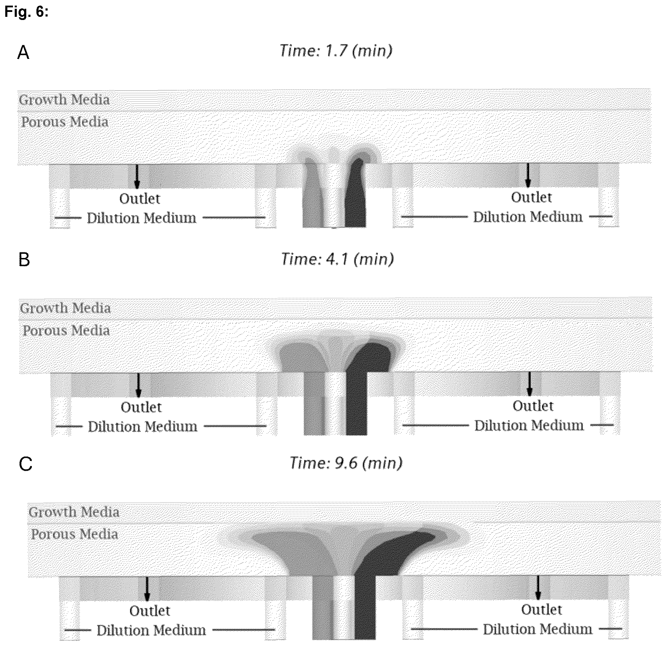

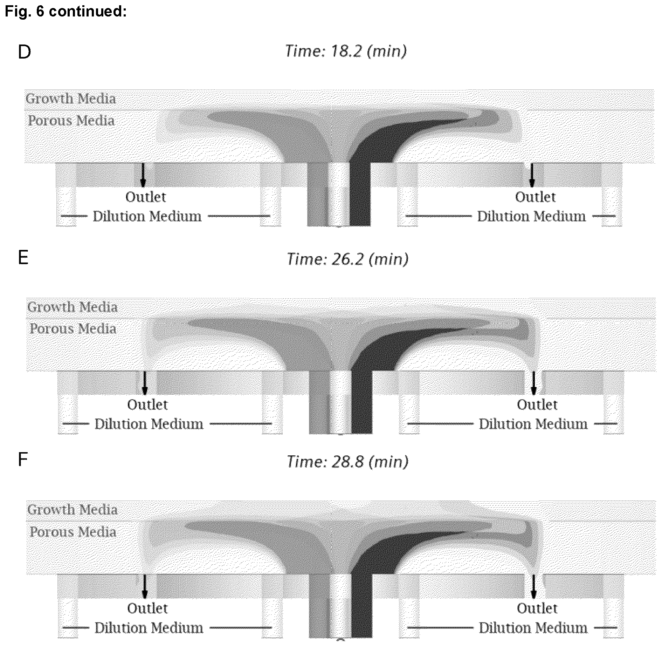

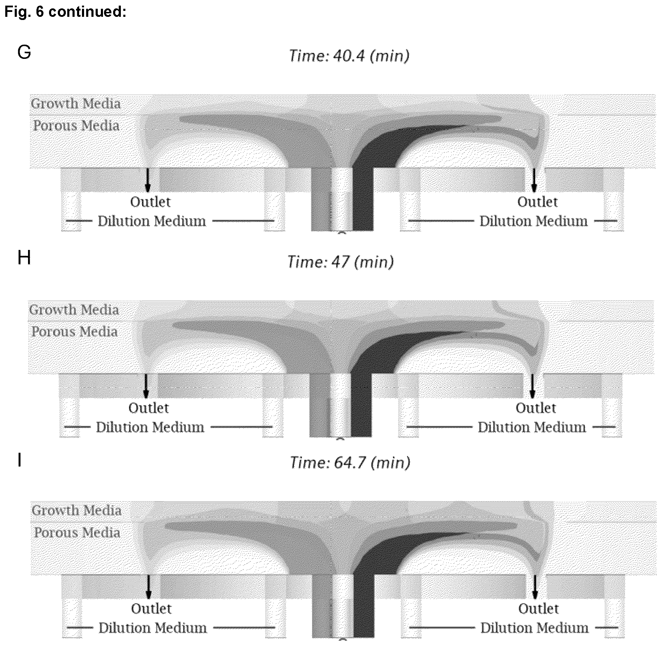

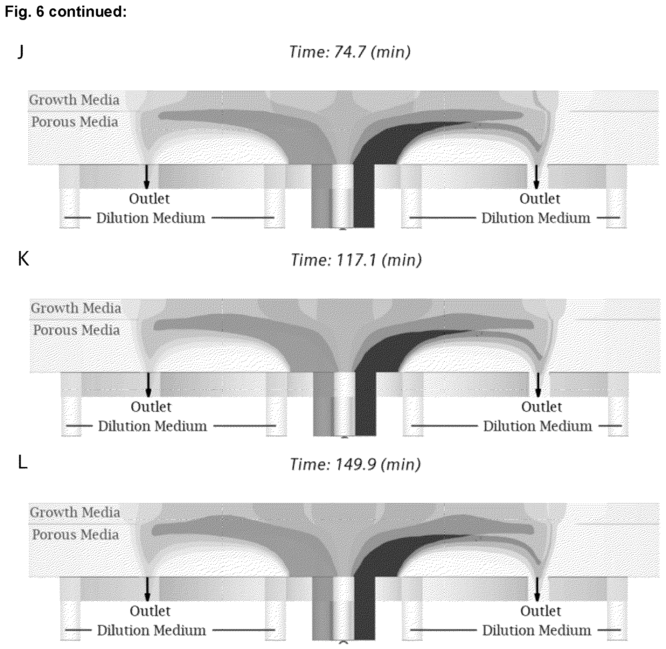

The principle of the concentration gradient generation is that one or more fluids, each comprising a biologically active compound, or mixtures of two or more biologically active compounds, can be injected into the reactor. The fluids (if more than one is used) mix in the second layer, preferably by convection. In embodiments, the fluid(s) and the dilutant are injected simultaneously. In alternative embodiments, the fluid(s) and the dilutant are injected sequentially, wherein either the fluid(s) are injected first or the dilutant is injected first. In embodiments, the dilutant "guides" or "pushes" the fluids towards the third layer and dilutes the fluids. This function is fulfilled by the dilutant streaming in from the inner dilutant inlet(s). In embodiments, the dilutant streaming in from the outer dilutant inlet(s) limits the space to which the fluids extend and may also, in embodiments, dilute the fluids, so that the gradient generation is largely limited to the area within and restricted by the outlets (see also **Fig. 6**). Outside of the area restricted by the outlets, microorganisms and cells may grow without the presence of compound(s), which may, in embodiments, represent a (growth/proliferation starting) zone in which the microorganisms or cells can grow and/or proliferate without being restricted by the biologically active compound. If only one fluid is injected, only the dilutant inlets and the outlet on the side of the used fluid inlet may be needed in the methods according to the invention (see also **Fig. 1C**).

When the concentration gradient is established, or while it is formed, the concentration gradient can, in embodiments, transfer from the second layer to the third layer, preferably by diffusion. In preferred embodiments, the third layer is so thin (e.g. only about 1 mm) that the concentration gradient transfers without loss, or without substantial loss, of compound concentration into the direction of the diffusion. This is an advantage over systems that are based on fluid stream-based gradient generation in which fluids are lead past a culture medium.

In embodiments, the time needed to build the concentration gradient(s) ranges from 10 min to 24 hours, preferably from 12 min to 20 hours, more preferably from 15 min to 12 hours, even more preferably from 17 min to 8 hours, even more preferably from 20 min to 2.5 hours.

The time to build the concentration gradient depends on the diffusion properties of the fluid(s) comprising the biologically active compound and the diffusion properties of the dilutant.

In embodiments, the time needed to build the concentration gradient(s) in the third layer ranges from 10 min to 2 hours, or from 20 min to 1.5 hours, or from 30 min to 1 hour, per mm of the third layer.

The invention further relates to a method for detecting an influence of at least one biologically active compound on the growth, proliferation and/or survival of microorganisms or cells, comprising:
- providing a gradient reactor according to the invention, or a system according to the invention,
- simultaneously injecting the dilutant and the at least one fluid, into the at least one dilutant inlet and the at least one fluid inlet, respectively, wherein each of the at least one fluids comprises a different biologically active compound, wherein the different biologically active compounds influence or are suspected to influence growth, proliferation and/or survival of microorganisms or cells,
- generating one or more concentration gradients for the different biologically active compounds in the second layer,
- wherein the one or more concentration gradients transfer from the second layer to the third layer, wherein the third layer comprises the microorganisms or cells,
- wherein an influence on the growth, proliferation and/or survival of the microorganisms or cells is analyzed.

In embodiments, the diffusion constant is calculated or known for the tested compounds, wherein preferably, said calculated diffusion constant enables the numerical pre-simulation of the gradient propagation/formation such that every desired gradient can automatically be generated by the reactor.

In embodiments, the reactor, system and methods of the invention can be used for diagnostic purposes (e.g. susceptibility testing or identification of resistant microorganisms or cells), environmental monitoring, or pharmaceutical development of compounds.

In embodiments, the reactor and system and methods of the invention provide a patient-specific point-of-care system for rapid combination therapy of infections, e.g., bacterial, viral or fungal infections.

In embodiments, the reactor, system and methods of the invention can be used to experimentally test antibiotic resistance evolution for new treatment regimens and new drugs before application. Thus, antibiotics, for example, can be used sustainably, the spread of antimicrobial resistance (AMR) genes and organisms can be reduced, and persistent infections can be treated more efficiently.

It is an advantage of the reactor, system, and methods according to the invention, that different compounds, dilutions and/or mixtures thereof can be tested at once, and due to the resulting overlapping concentration gradients, a large amount of different concentrations and combinatorial combinations (with a certain resolution, (almost) all concentration combination gradations) can be tested. It is further an advantage of the reactor, system, and methods according to the invention, that i) the desired concentration gradients can be generated quickly, for example, within 2 hours, in the third layer, ii) the gradient is dynamically adaptable, iii) they can be automated, and iv) the desired concentration gradient can be simulated.

It is a further advantage of the reactor, system, and methods according to the invention, that convection and diffusion can both be employed, thus enabling a (fast) generation of stationary concentration gradients, while also enabling a quick switch between different compounds to be tested and/or their concentrations due to dynamic concentration gradient generation. For the stationary gradient generation, a constant supply of the fluids and dilutant is provided, at least until the gradient is fully formed. If the gradient shall be changed or if other compounds or combinations shall be tested, i.e. if a dynamic gradient is required, the fluid supply can be stopped and dilutant can be injected into the fluid inlets. Due to the low (or lack of) compound concentration in the dilutant, the compounds will diffuse from the third layer to the second layer and will be ejected or will drain from the outlets. Subsequently, a new concentration gradient can be generated. In alternative embodiments, a dynamic gradient is produced by varying the concentration of the injected fluid(s). This results in a shift of the concentration distribution and a change of the concentration gradient at the surface of and/or in the third layer, so that, in embodiments, different and/or changing compound concentrations affect the microorganisms or cells. Hence, in embodiments, the reactor and methods according to the invention can be used for stationary and/or dynamic concentration gradient generation. Gradients can thus not only be generated localized, but also temporarily. In embodiments, the different concentration gradients overlap to some degree. Based on the knowledge of the concentration of the compounds in the different fluids and the flow behavior of the fluids and the dilutant, the concentration of each compound on each point of the third layer can be computed. Based on this information, conclusions on the minimum inhibitory concentration, pharmacokinetics etc. can be drawn.

### Definitions

As used herein, the term "reactor" and "gradient reactor" are used interchangeably.

As used herein, the term "gradient" and "concentration gradient" are used interchangeably.

As used herein, the term "compound" and "biologically active compound" are used interchangeably.

A "fluid" as used herein refers to fluids comprising a biologically active compound.

A "torus-shaped structure" means a structure having the shape of a torus or donut, but may also refer to shapes in which the surface of the "torus" has corners, wherein the corners, for example, have about 90°, and optionally wherein the surface comprises one or more substantially planar surfaces.

"The minimum inhibitory concentration" or "MIC" as used herein is the lowest concentration of a chemical, usually a drug, which prevents visible in vitro growth of bacteria or fungi.

"Biocides" as used herein refers to a diverse group of poisonous substances including preservatives, insecticides, disinfectants, and pesticides used for the control of organisms that are harmful to human or animal health or that cause damage to natural or manufactured products. Examples include pesticides, such as fungicides; herbicides; insecticides; algicides; molluscicides; miticides; piscicides; rodenticides; and slimicides, and antimicrobials, such as germicides; antibiotics; antibacterials; antivirals; antifungals; antiprotozoals; and antiparasitics, and spermicides.

"Stressors" as used herein refers to a chemical or biological agent causing stress to an organism. Examples include disinfectants, salt, alcohol, drugs.

In general, the features disclosed in the context of the gradient reactor, or the system, or a method, or the use of the reactor or system of the invention also relate to and are herewith disclosed in the context of any of the other ones of the gradient reactor, or the system, or a method, or the use of the reactor or system of the present invention and *vice versa.*

### FIGURES

The invention is demonstrated by way of example through the figures disclosed herein. The figures provided represent particular, non-limiting embodiments and are not intended to limit the scope of the invention.
**Figure 1****:**
   **A)** Schematic cross-sectional view of the gradient reactor according to the invention.
   **B)** Schematic cross-sectional view of the gradient reactor according to the invention showing two centrally located inlets (in this case serving as inlets for exemplary antibiotic A and B, respectively) and one further centrally located inlet through which a mixture of antibiotic A and B can be injected. A further centrally located inlet, which is not visible as it is located behind the other three centrally located inlets, can also be used for injection of a mixture of antibiotic A and B. The two dilutant inlets next to the centrally located inlets are connected via a torus-shaped structure as indicated in subfigure A. Similarly, the outer two dilutant inlets and the two outlets are connected via torus-shaped structures, respectively.
   **C)** Sectional view of the numerical simulation of the combination approach of diffusion and convection at different time points of the gradient formation. Different coloring of the concentration ratio of one of the substances.

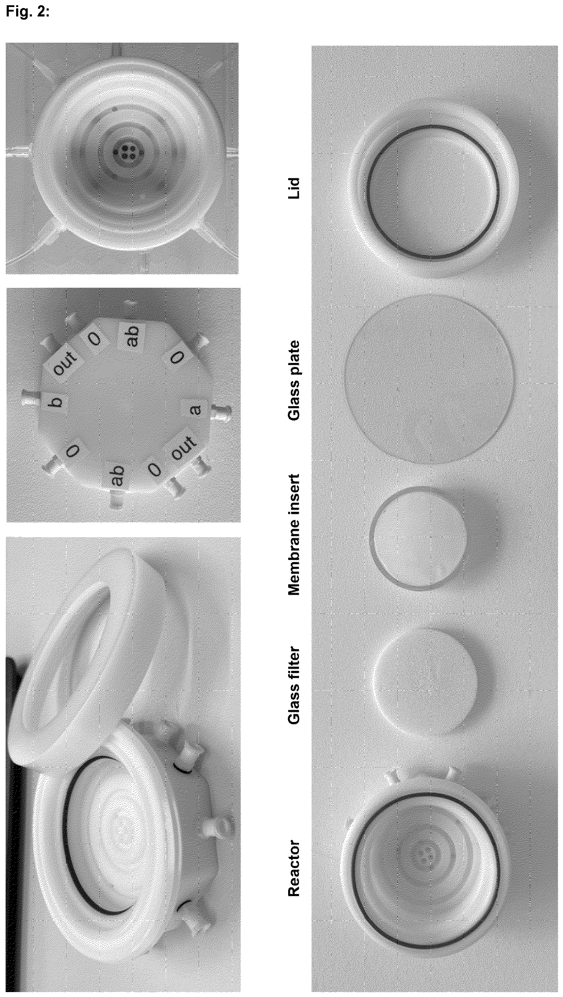

**Figure 2****:** Structure of the assembled (top panels) and disassembled (bottom panels) gradient reactor according to the invention.

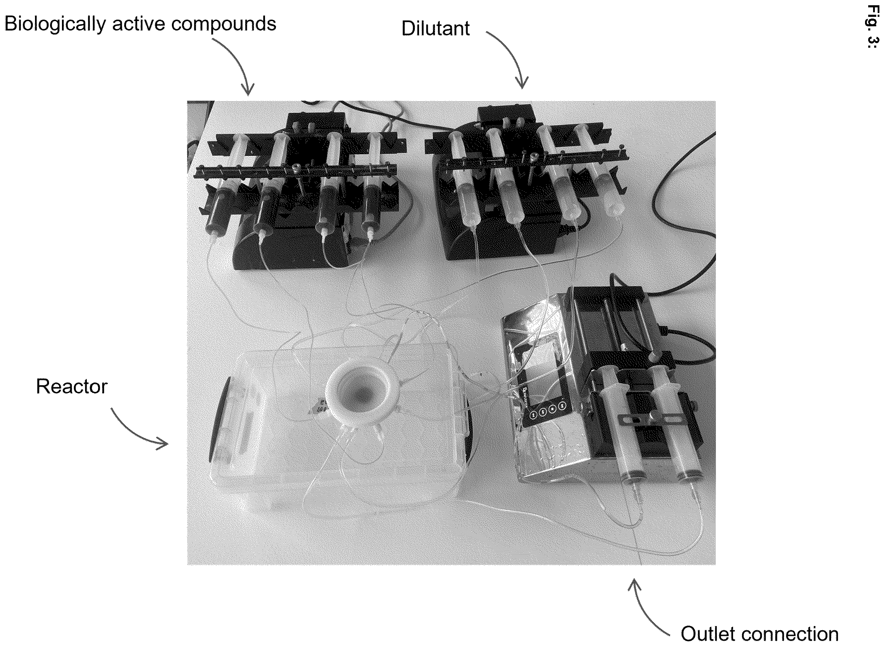

**Figure 3****:** Structure of the system according to the invention. In this exemplary assembly, the reactor is connected to syringes used for fluid injection and ejection.

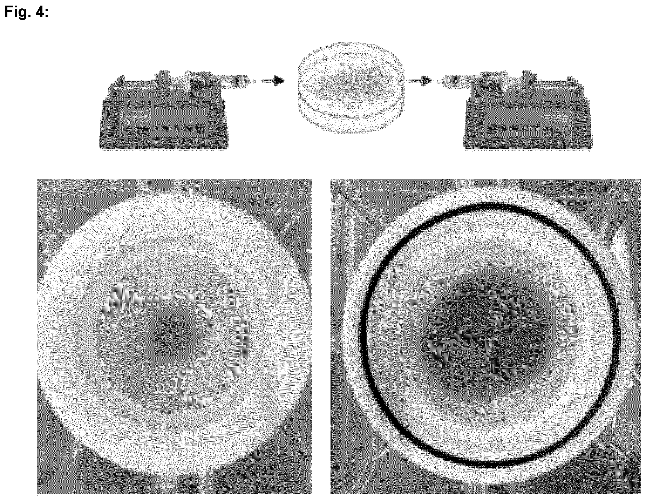

**Figure 4****:** Concentration gradient formation in an embodiment of the reactor without a third layer, wherein the concentration gradient is only formed in the second layer (in this example, the second layer is a glass filter). **Upper panel:** a schematic representation of microorganism growth in particular regions of the growth medium in dependence of the concentration of the biologically active compounds. **Bottom left:** the picture was taken a few seconds after injection of the fluids. **Bottom right:** the picture was taken one minute after injection of fluids.

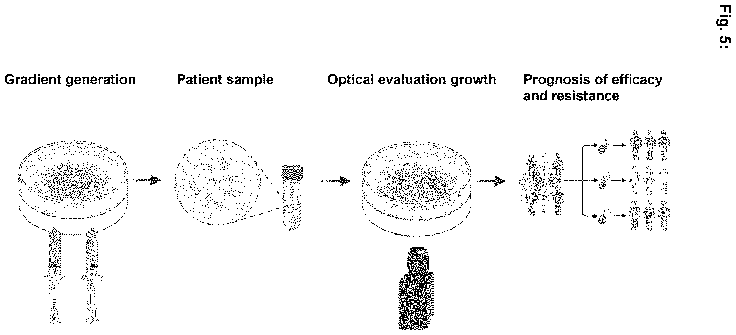

**Figure 5****:** Workflow of an embodiment of the method of the invention. A patient sample is obtained and the microorganism(s) comprised therein are inoculated and incubated on a suitable growth medium in the reactor according to the invention. Concentration gradients for two different biologically active compounds are generated and the effect on the microorganisms is detected and analyzed. A prognosis of the efficacy and of the resistance can be made based on the results of the analysis.
**Figure 6****:** Sectional view of the reactor depicting an exemplary simulation of the combination approach of convection (in second layer, here: "porous media") and diffusion (into the third layer, here: "growth media") at different time points of the gradient formation (**A-L**). The inlet in the center, which is sandwiched by the inlets through which two different biologically active compound-comprising fluids are injected, comprises a mixture of the two different exemplary biologically active compound-comprising fluids.

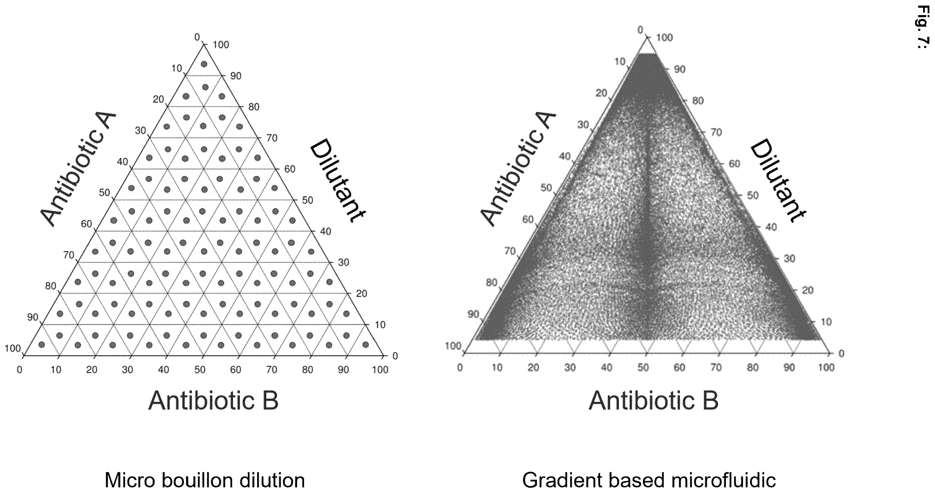

**Figure 7****:** Ternary phase diagram to illustrate the expected concentration and combination gradations in the reactor. Comparison of 100 direct combinations by pipetting mixtures in a microbouillon dilution and the coverage of the concentration range in the nutrient medium layer of the reactor. The data points were taken from the numerical simulation.

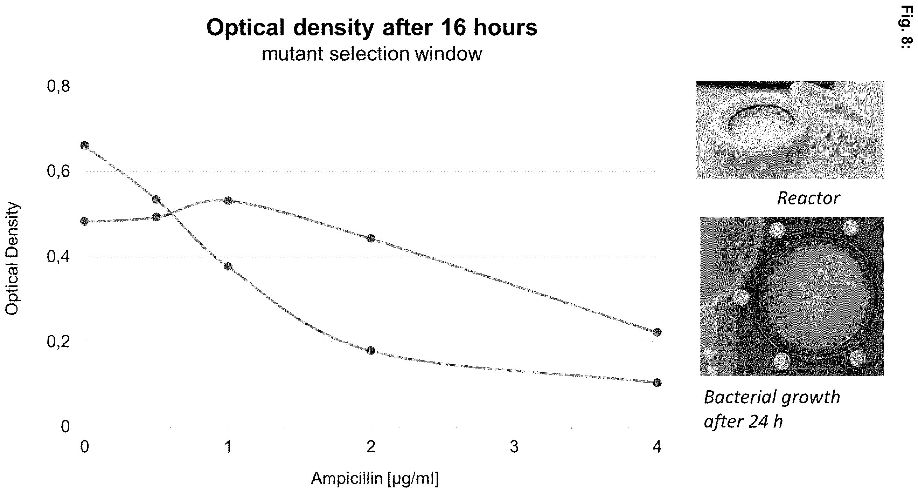

**Figure 8****:** Optical density of *E.coli MG1655* and a sample from an area with a high antibiotic concentration during inoculation with 4 µg/ml Ampicillin for 16 hours.

### EXAMPLES

The invention is demonstrated through the examples disclosed herein. The examples provided represent particular embodiments and are not intended to limit the scope of the invention. The examples are to be considered as providing a non-limiting illustration and technical support for carrying out the invention.

### Example 1:

The present invention allows statements about the effectiveness of a particular compound concentration and an assessment of the probability of future resistances. Experiments were able to demonstrate resistance evolution of *E.coli MG1655* against Ampicillin within the reactor. The detected MIC of the sample had a rise in MIC from 2 µg/ml to 4 µg/ml of Ampicillin. See **Fig. 8****.**

## Claims

1. A gradient reactor for generating a concentration gradient, comprising:
- a first layer comprising
i) at least one fluid inlet for an injection of at least one fluid,
ii) at least one dilutant inlet for an injection of a dilutant, and
iii) at least one outlet for an ejection of the at least one fluid,
- a second layer,
- a third layer suitable for culturing microorganisms or cells,
wherein a concentration gradient is formable in the second layer, and the concentration gradient is transferable from the second layer to the third layer.

2. The gradient reactor according to claim 1, wherein the second layer comprises a material selected from the group consisting of i) a porous material, such as a glass filter, ii) stainless steel, iii) 3D-printed material, iv) a material comprising microchannels, and v) combinations thereof.

3. The gradient reactor according to any one of the preceding claims, wherein the third layer has a thickness of 0.05-10 mm, preferably 0.1-5 mm.

4. The gradient reactor according to any one of the preceding claims, wherein each of the at least one fluids comprises a different biologically active compound, wherein the different biologically active compounds influence or are suspected to influence growth, proliferation and/or survival of microorganisms or cells, preferably wherein the third layer comprises the microorganisms or cells.

5. The gradient reactor according to claim 4, wherein the different biologically active compounds are selected from the group consisting of
i) antimicrobial agents, such as antibiotic, antiviral, antifungal, or antiparasitic agents,
ii) biocides,
iii) stressors,
and
iv) drugs, such as chemotherapeutic agents.

6. The gradient reactor according to any one of the preceding claims, wherein the concentration gradient is transferable from the second layer to the third layer by diffusion.

7. The gradient reactor according to any one of the preceding claims, wherein the dilutant is selected from the group consisting of water and medium.

8. The gradient reactor according to any one of the preceding claims, wherein at least a portion of the at least one fluid inlet is arranged substantially perpendicularly to a surface of the second layer and towards a center of the surface of the second layer.

9. The gradient reactor according to any one of the preceding claims, wherein the at least one fluid inlet is four fluid inlets arranged to form the corners of a square.

10. The gradient reactor according to claim 8 or 9, wherein one of the at least one dilutant inlets is arranged between the at least one fluid inlet and the at least one outlet.

11. The gradient reactor according to any one of the preceding claims, wherein the concentration gradient is a stationary or dynamic concentration gradient.

12. A system comprising
- the gradient reactor according to any one of the preceding claims, wherein the gradient reactor optionally further comprises pumps, preferably micropumps, for automated injection of the at least one fluid and the dilutant,
- a camera system, and
- a computer program comprising instructions, optionally wherein the instructions, when the program is executed by a computer, cause the computer to analyze the growth, proliferation and/or survival of the microorganisms or cells.

13. The system according to claim 12, wherein the analysis of the growth, proliferation and/or survival of the microorganisms or cells comprises the computation of the effective concentration of the different biologically active compounds.

14. Use of the gradient reactor according to any one of the preceding claims, for the generation of overlapping stationary or dynamic concentration gradients, for analyzing the efficacy or effect of different biologically active compounds, for assessing possible combinatory therapies, for assessing the probability of future resistance to the different biologically active compounds, or for analyzing pharmacokinetics.

15. A method for generating one or more concentration gradients, comprising:
- providing a gradient reactor according to any one of claims 1 to 11, or a system according to any one of claims 12 to 13,
- simultaneously injecting the dilutant and the at least one fluid, into the at least one dilutant inlet and the at least one fluid inlet, respectively,
- generating one or more concentration gradients in the second layer,
- wherein the one or more concentration gradients transfer from the second layer to the third layer, wherein the third layer is suitable for culturing microorganisms or cells.
